Europäisches Patentamt

(19) European Patent Office

Office européen des brevets

(11) Publication number : **0 443 047 B1**

(12) **EUROPEAN PATENT SPECIFICATION**

(45) Date of publication of patent specification :
23.03.94 Bulletin 94/12

(51) Int. Cl.⁵ : **A23L 1/29,** A23L 1/30,
A23L 1/305, A23L 1/09

(21) Application number : 90913552.7

(22) Date of filing : 13.09.90

(86) International application number :
PCT/JP90/01173

(87) International publication number :
WO 91/03948 04.04.91 Gazette 91/08

(54) **LIQUID NUTRIENT COMPOSITION.**

(30) Priority : 14.09.89 JP 239524/89

(43) Date of publication of application :
28.08.91 Bulletin 91/35

(45) Publication of the grant of the patent :
23.03.94 Bulletin 94/12

(84) Designated Contracting States :
DE FR IT NL

(56) References cited :
EP-A- 0 164 656
EP-A- 0 242 459
EP-A- 0 246 747
JP-A- 1 240 169
JP-B- 6 342 510
WORLD PATENTS INDEX LATEST Section Ch,
Week 8330, Derwent Publications Ltd., Lon-
don, GB; Class D, AN 83-71972 & JP-A-58 101
700 (SNOW BRAND MILK PRODUCTS) 16 June
1983
WORLD PATENTS INDEX LATEST Section Ch,
Week 8837, Derwent Publications Ltd., London, GB; Class D, AN 86-11593 & JP-B-63 042
510 (NIPPON OILS & FATS) 24 August 1988

(73) Proprietor : OTSUKA PHARMACEUTICAL CO.,
LTD.
9, Kandatsukasa-cho 2-chome
Chiyoda-ku Tokyo 101 (JP)

(72) Inventor : TAKAICHI, Akihisa 172-3, Aza
Nakajima Takashima
Narutocho
Naruto-shi Tokushima 772 (JP)
Inventor : AZUMA, Yoshihide 701,
Mezon-Kitahama
21-9, Aza Miyanohigashi Kitahama
Muyacho, Naruto-shi Tokushima 772 (JP)
Inventor : OTSUKA, Ichiro 13-1, Aza Fukuike
Tosadomariura
Narutocho
Naruto-shi Tokushima 772 (JP)

(74) Representative : Hansen, Bernd, Dr.
Dipl.-Chem. et al
Hoffmann, Eitle & Partner Patent- und
Rechtsanwälte, Postfach 81 04 20
D-81904 München (DE)

## Description

FIELD OF UTILIZATION

The present invention relates to a nutrient fluid composition, particularly a high-protein, high-calorie fluid composition containing nutrients in a good balance suited for alimentation, and a method for producing said fluid composition.

BACKGROUND TECHNOLOGY

Dietary life, particularly management of the nutritional state, is of paramount importance for maintenance of health. Omission of breakfasts and untimely lunches or suppers, for instance, not only frustrate the feeding of energy and nutrients necessary for activities of daily living but also disturb the rhythm of physiology to suppress various functions of the body, exert undue loads on the various organs and even lead to obesity which is one of the fundamental causes of adult diseases such as diabetes, hypertension, heart disease and so on. Therefore, a diversity of liquid or solid preparations containing a variety of nutrients required for activities of daily living are already available for use as supplements to meals or as sources of nutrients for quick replenishment of the energy consumed in sports and work. However, all of them have the crucial disadvantage that they are generally lean in protein. Furthermore, these nutrient preparations are not fully satisfactory, quality-wise and quantity-wise, particularly in the balance of nutrients, the ease of digestion and absorption, and nourishing value.

WPIL, Section Ch, Week 8837, Derwent Publications, Ltd., London, GB, Class D, AN 86-115934 & JP-B-63042510 reveals a nutritive composition including protein or its decomposed products, sugar, lipids, vitamins, minerals and water, which may furthermore include an emulsifier. The above-disclosed composition is not specified with regard to the amounts of the ingredients and the viscosity and caloric values.

EP-A-0 246 747 discloses a dietary composition comprising 5 g/100 ml protein and enzymatically hydrolyzed protein in a ratio of 1:1, 11.89 g/100 ml maltodextrin and 2.98 g/100 ml fat, and having a caloric value of 419kJ (100 kcal)/100 ml.

The object of the present invention is to provide a novel nutrient fluid composition free from the above-mentioned disadvantages of the known nutrient preparations and a method for producing said novel fluid composition.

To accomplish the above-mentioned object, the inventors of the present invention explored these and other problems in the art and found that a fluid composition having the formulational features and properties described hereinafter are quite effective in accomplishing the above-mentioned object, enables improvements in unbalanced dietary life, insures proper supply of the energy and nutrients necessary for daily activity and, as a corollary, contributes to the prophylaxis and treatment (prevention of exacerbation) of a variety of diseases due to excessive intake of energy and consequent obesity, such as diabetes, hypertension, heart disease and so on. The present invention has been conceived and developed on the basis of the above finding.

DISCLOSURE OF THE INVENTION

In accordance with the present invention, there is provided a nutrient fluid composition comprising 3.5 to 7 g of total protein, 5 to 17 g of carbohydrate and 1 to 5 g of fat in each 100 milliliters, with a water-soluble enzymatically degraded protein having a molecular weight of 800 to 30,000 accounting for 30 to 50 weight percent of said total protein and one or more oligosaccharides selected from the group consisting of maltotriose, maltotetrose, maltopentose and maltohexose accounting for 50 to 100 weight percent of said carbohydrate, and said composition having a viscosity [as measured with a Type B viscosimeter; the same applies hereinafter] of not more than 7 mPa·s (7 cps) at 20°C and a total caloric value of 293 to 544 kj (70 to 130 kcal)/100 ml.

The invention further provides a method for producing the above nutrient fluid composition which comprises mixing and emulsifying the components thereof in water.

Because of the above formulation and properties, the composition of the present invention is not only easy to ingest (drink) but also provides sufficient nutrients and energy, particularly protein and calorie, on ingestion.

Moreover, the composition of the invention is well-balanced in the assortment of nutrients which, coupled with the above-mentioned protein and energy supplementation effect, produces prophylactic or aggravation-preventive effects on obesity and adult diseases. Particularly, the nutrient fluid composition of the present invention is suited for use as a breakfast food capable of providing a vital force needed for the day or as a convenient alimentation means for people with irregular eating habits, poor appetite or inability to take sufficient meals. The composition can also be utilized as a snack food for growing children and elderly persons, and even for energy supplementation after sporting exercises or as an emergency food.

The nutrient fluid composition of the present invention can be manufactured by the per se conventional procedure for production of nutrient foods of this kind except that the formulation of its components and the properties of the product are controlled as specified herein.

The protein as a component of the nutrient fluid composition of the present invention may be in the form of any known protein source only if it contains said enzymatically degraded protein in a specified proportion. The said protein source includes, among others, non-heat-coagulable proteinous materials such as casein and its salts such as casein sodium, casein calcium, etc. and enzymatic degradation products thereof, enzymatic degradation products of soybean protein, wheat protein and so on. These protein sources can be used independently or in combination. The above-mentioned enzymatic degradation products of protein should be selected from among water-soluble proteins with molecular weights in the range of 800 to 30,000, preferably 8,000 to 30,000 and more preferably 10,000 to 15,000. As examples of such water-soluble protein, there may be mentioned enzymatically hydrolyzed gelatin (water-soluble gelatin) and enzymatically hydrolyzed casein. These degraded proteins are used in a proportion of 30 to 50 weight %, preferably 35 to 46 weight percent, of the total protein.

The molecular weight of such enzymatically degraded protein is expressed in the maximum peak value found from the molecular weight distribution and concentration distribution determined by gel filtration chromatography and SDS-PAGE, respectively.

By utilizing such enzymatic degradation-product of protein, the adverse effect of increased viscosity on the palatability of the composition can be obviated even if the total protein concentration is high. Moreover, in cases where the use of a water-soluble gelatin, for instance, might detract from the protein score, this drawback can be obviated, and a sufficient nourishing effect can be sustained, by using a high quality protein such as casein in conjunction.

The carbohydrate as a component of the nutrient fluid composition of the present invention may be any known carbohydrate source only if it contains one or more oligosaccharides selected from the group consisting of maltotriose, maltotetrose, maltopentose and maltohexose in a proportion of 50 to 100 weight %, preferably 70 to 100 weight %. Preferred are tetra- and pentasaccharides, such as maltotetrose, maltopentose and so on. The use of such oligosaccharides is advantageous in that the tendency of increased osmotic pressure and excessive sweetness can be prevented. The carbohydrate includes, inter alia, monosaccharides, oligosaccharides and polysaccharides, such as glucose, maltose, sucrose, isomaltose, maltotriose, maltotetrose, maltopentose, maltohexose, lactose, glycogen, dextrin, starch and so on.

The fat as a component of the nutrient fluid composition of the present invention may be any known fat source, whether of aninal origin or of vegetable origin, such as rice oil, cottonseed oil, corn oil, soybean oil, sunflower oil, cacao butter, sesame oil, safflower oil, peanut oil, butter, lard, coconut oil, nut oil, palm oil, rapeseed oil and so on. Particularly preferred are vegetable oils.

It is essential that the nutrient fluid composition of the present invention contain said protein, carbohydrate and fat in the herein-specified proportions. Thus, these components are incorporated in proportions such that each 100 milliliter of the fluid composition will contain 3.5 to 7 g of total protein, 5 to 17 g of carbohydrate and 1 to 5 g of fat and preferably 4.5 to 7 g of total protein, 10 to 16 g of carbohydrate and 2 to 3 g of fat.

Where necessary, the nutrient fluid composition of the present invention may further contain a variety of additives which are commonly employed. Among such additives are various vitamins (e.g. vitamin A, vitamin $B_1$, vitamin $B_2$, vitamin $B_6$, vitamin $B_{12}$, vitamin C, vitamin D, vitamin E, niacin, folic acid, pantothenic acid, etc.), various minerals (e.g. calcium, iron, potassium, sodium, magnesium, phosphorus, chlorine and other salts), various flavors such as synthetic and natural flavors, sweeteners inclusive of natural sweeteners (thaumatin, stevia, etc.) and synthetic sweeteners (saccharin, cyclamate, etc.), colors, emulsifiers, stabilizers, preservatives and so on. These additives can be used singly or in combination.

The composition of the present invention is manufactured by mixing and emulsifying the above-mentioned components and the manufacturing process as such is not particularly limited. Generally, however, the preferred procedure comprises dissolving the water-soluble materials such as the carbohydrate and protein in water or hot water, heating the solution, then adding the liposoluble material such as the fat, and emulsifying the mixture in an appropriate homogenator.

The thus-obtained composition of the present invention is aseptically filled into suitable containers, or filled into cans, retort pouches or the like and heat-sterilized in the routine manner for marketing.

The emulsion obtained as above should have a viscosity, at 20°C, of not more than 7 cps, preferably not more than 6 mPa·s (6 cps), and for still better results, not more than 5 mPa·s (5 cps), and a caloric value of 293 to 544 kJ (70 to 130 kcal)/100 ml. As such, the composition of the invention is of value as a high concentration, high energy turnover alimentation fluid.

The composition of the present invention is a liquid preparation which is not only rich in protein and energy and well-balanced in the assortment of nutrients as mentioned above but, after ingestion, is efficiently decomposed (digested) and absorbed from the testinal tract and constantly insures improvements in the nutritional state.

Examples

The following examples of the production of nutrient fluid compositions are further illustrative of the present invention. The protein contents of the protein sources and the sugar contents of the carbohydrate sources used in the examples are set forth below.

| Protein source | Protein content (%) |
|---|---|
| Casein | 87 |
| Casein sodium | 92 |
| Casein calcium | 92 |
| Enzymatically degraded casein | 92 |
| Enzymatically degraded soybean | 92 |
| Enzymatically degraded gelatin | 95 |

| Carbohydrate source | Sugar content (%) |
|---|---|
| Dextrin* | 67 |
| Maltotetrose syrup | 75 |
| Maltotriose syrup | 75 |
| Maltose syrup | 75 |

The dextrin* contains tri- to hexa-saccharides in a proportion of 50 weight % of total carbohydrate.

Example 1

The component materials in amounts (g/100ml) sufficient to give the compositions shown in Table 1 were mixed and emulsified in water. The resulting nutrient fluid compositions of the present invention were respectively filled into containers and heat-sterilized.

Table 1

| Formulation No. | 1 | 2 | 3 | 4 | 5 |
|---|---|---|---|---|---|
| Protein (g/100 ml) | 5.2 | 6.8 | 5.7 | 5.0 | 5.6 |
| Carbohydrate (g/100 ml) | 15.0 | 15.5 | 11.9 | 12.6 | 9.8 |
| Fat (g/100 ml) | 2.2 | 2.3 | 2.2 | 2.2 | 2.1 |
| Energy kj(kcal) | 419(100) | 460(110) | 377(90) | 377(90) | 335(80) |
| Protein composition | | | | | |
| Casein | 3.3 | 4.5 | – | – | – |
| Casein sodium | – | – | 2.2 | 2.6 | 3.3 |
| Casein calcium | – | – | 1.1 | 0.6 | – |
| Enzymatically degraded casein | – | – | 0.7 | 2.2 | – |
| Enzymatically degraded soybean protein | – | – | 0.2 | – | 0.5 |
| Enzymatically degraded gelatin | 2.5 | 3.1 | 2.0 | – | 2.2 |
| Sugar composition | | | | | |
| Dextrin | – | – | 8.4 | – | – |
| Maltotetrose syrup | 20.0 | 14.9 | – | 10.3 | – |
| Maltotriose syrup | – | 5.8 | 8.4 | – | 13.1 |
| Maltose syrup | – | – | – | 6.5 | – |
| Fat composition | | | | | |
| Soybean oil | 2.0 | – | – | – | – |
| Rice oil | – | 2.3 | – | 1.2 | – |
| Cottonseed oil | – | – | 2.2 | – | 1.5 |
| Peanut oil | – | – | – | 1.0 | – |
| Macadamia nut oil | 2.0 | – | – | – | 0.6 |
| Other components | | | | | |
| Vitamins | q.s. | q.s. | q.s. | q.s. | q.s. |
| Minerals | q.s. | q.s. | q.s. | q.s. | q.s. |
| Flavor | q.s. | q.s. | q.s. | q.s. | q.s. |

EP 0 443 047 B1

Table 1 (Continued)

| Formulation No. | 6 | 7 | 8 | 9 | 10 |
|---|---|---|---|---|---|
| Protein (g/100 ml) | 6.9 | 5.0 | 3.5 | 5.1 | 5.0 |
| Carbohydrate (g/100 ml) | 8.0 | 8.7 | 17.0 | 12.6 | 8.7 |
| Fat (g/100 ml) | 3.4 | 1.7 | 2.0 | 2.2 | 1.7 |
| Energy kJ (kcal) | 377(90) | 293(70) | 419(100) | 377(90) | 293(70) |
| Protein composition | | | | | |
| Casein | - | - | - | - | - |
| Casein sodium | 4.0 | 2.9 | 2.6 | 3.2 | 3.3 |
| Casein calcium | - | - | - | - | - |
| Enzymatically degraded casein | 0.6 | 0.4 | 1.2 | - | - |
| Enzymatically degraded soybean protein | 0.2 | - | - | 0.3 | - |
| Enzymatically degraded gelatin | 2.6 | 2.1 | - | 2.0 | 2.1 |
| Sugar composition | | | | | |
| Dextrin | 3.1 | - | - | - | 13.0 |
| Maltotetrose syrup | - | 6.0 | 22.7 | 16.8 | - |
| Maltotriose syrup | 8.0 | - | - | - | - |
| Maltose syrup | - | 5.6 | - | - | - |
| Fat composition | | | | | |
| Soybean oil | 2.4 | · | - | 2.3 | - |
| Rice oil | 1.0 | 1.0 | 2.0 | - | - |
| Cottonseed oil | - | - | - | - | 1.7 |
| Peanut oil | - | - | - | - | - |
| Macadamia nut oil | - | 0.7 | - | - | - |
| Other components | | | | | |
| Vitamins | q.s. | q.s. | q.s. | q.s. | q.s. |
| Minerals | q.s. | q.s. | q.s. | q.s. | q.s. |
| Flavor | q.s. | q.s. | q.s. | q.s. | q.s. |

The above nutrient fluid compositions of the present invention were rich in protein and energy and well-balanced in nutrients and insured an improving effect on the nutritional state.

**Claims**

1. A nutrient fluid composition comprising 3.5 to 7 g of total protein, 5 to 17 g of carbohydrate and 1 to 5 g of fat in each 100 milliliters, with a water-soluble enzymatically degraded protein having a molecular weight of 800 to 30,000 accounting for 30 to 50 weight percent of said total protein and one or more oligosaccharides selected from the group consisting of maltotriose, maltotetrose, maltopentose and maltohexose accounting for 50 to 100 weight percent of said carbohydrate, and said composition having a viscosity of not more than 7 mPa·s (7 cps) at 20°C and a total caloric value of 293 to 544 kj (70 to 130 kcal)/100 ml.

2. The composition of claim 1 which contains 4.5 to 7 g of total protein, 10 to 16 g of carbohydrate and 2 to 3 g of fat in each 100 millliters.

3. The composition of claim 1 wherein said water-soluble protein having a molecular weight of 8,000 to 30,000 accounts for 35 to 46 weight percent of said total protein and one or more oligosaccharides selected from the group consisting of maltotriose, maltotetrose, maltopentose and maltohexose accounting for 70 to 100 weight percent of said carbohydrate.

4. The composition of claim 3 wherein said water-soluble protein has a molecular weight in the range of 10,000 to 15,000 and said carbohydrate is one or more oligo(tetra-/penta-)saccharides selected from the group consisting of maltotetrose and maltopentose.

5. The composition of claim 1 which contains 4.5 to 7 g of total protein, 10 to 16 g of carbohydrate, and 2 to 3 g of fat in each 100 milliliters and in which said water-soluble protein having a molecular weight of 8,000 to 30,000 accounts for 35 to 46 weight percent of said total protein and one or more oligosaccharides selected from the group consisting of maltotriose, maltotetrose, maltopentose and maltohexose account for 70 to 100 weight percent of said carbohydrate.

6. The composition of claim 1 which contains 4.5 to 7 g of total protein, 10 to 16 g of carbohydrate and 2 to 3 g of fat in each 100 milliliters and in which a water-soluble protein having a molecular weight in the range of 10,000 to 15,000 accounts for 35 to 46 weight percent of said total protein and one or more oligo(tetra/penta-)saccharides selected from the group consisting of maltotetrose and maltopentose account for 70 to 100 weight percent of said carbohydrate.

7. The composition of claim 5 which has a viscosity of not more than 5 mPa·s (5 cps) at 20°C.

8. The composition of claim 6 which has a viscosity of not more than 5 mPa·s (5 cps) at 20°C.

9. The composition of any of claims 1 through 8 wherein said protein is selected from the group consisting of casein, casein sodium, casein calcium, enzymatic degradation products of said caseins, enzymatically degraded soybean protein, enzymatically degraded wheat protein and enzymatically degraded gelatin, said carbohydrate is selected from the group. consisting of oligo(tetra-/penta-)saccharides, and said fat is selected from the group consisting of vegetable oils.

10. The composition of claim 9 wherein said oligosaccharide is maltotetrose and/or maltopentose.

11. A method of producing a nutrient fluid composition which comprises providing a protein source containing 30 to 50 weight percent of an enzymatically degraded protein having a molecular weight in the range of 800 to 30,000 and a carbohydrate source containing 50 to 100 weight percent of one or more oligosaccharides selected from the group consisting of maltotriose, maltotetrose, maltopentose and maltohexose, and a fat source and mixing and emulsifying said sources in water to give a fluid composition containing 3.5 to 7 g of total protein, 5 to 17 g of carbohydrate and 1 to 5 g of fat in each 100 milliliters, having a viscosity, at 20°C, of not more than 7 mPa·s (7 cps) and a total caloric value of 293 to 544 kJ (70 to 130 kcal)/100 ml.

## Patentansprüche

1. Flüssige Nährmittelzusammensetzung, umfassend 3,5 g bis 7 g Gesamtprotein, 5 bis 17 g Carbohydrat und 1 bis 5 g Fett in jeweils 100 ml, wobei ein wasserlösliches, enzymatisch abgebautes Protein mit einem Molekulargewicht von 800 bis 30.000 30 bis 50 Gew.-% des Gesamtproteins ausmacht, und ein oder mehrere Oligosaccharide, ausgewählt aus der Reihe Maltotriose, Maltotetrose, Maltopentose und Maltohexose, 50 bis 100 Gew.-% des Carbohydrats bilden, und wobei die Zusammensetzung eine Viskosität von nicht mehr als 7 mPa.s (7 cps) bei 20°C und einen Gesamtkalorienwert von 292 bis 544 kJ (70 bis 130 kcal)/100 ml aufweist.

2.  Zusammensetzung nach Anspruch 1, enthaltend 4,5 bis 7 g Gesamtprotein, 10 bis 16 g Carbohydrat und 2 bis 3 g Fett in jeweils 100 ml.

3.  Zusammensetzung nach Anspruch 1, wobei das wasserlösliche Protein mit einem Molekulargewicht von 8.000 bis 30.000 35 bis 46 Gew.-% des Gesamtproteins bildet, und ein oder mehrere Oligosaccharide, ausgewählt aus der Reihe Maltotriose, Maltotetrose, Maltopentose und Maltohexose, 70 bis 100 Gew.-% des Carbohydrats ausmachen.

4.  Zusammensetzung nach Anspruch 3, wobei das wasserlösliche Protein ein Molekulargewicht im Bereich von 10.000 bis 15.000 aufweist, und das Carbohydrat ein oder mehrere Oligo(tetra-/penta-)saccharide, ausgewählt aus der Reihe Maltotetrose und Maltopentose, darstellt.

5.  Zusammensetzung nach Anspruch 1, enthaltend 4,5 bis 7 g Gesamtprotein, 10 bis 16 g Carbohydrat und 2 bis 3 g Fett in jeweils 100 ml, und wobei das wasserlösliche Protein mit einem Molekulargewicht von 8.000 bis 30.000 35 bis 46 Gew.-% des Gesamtprotein bildet, und ein oder mehrere Oligosaccharide, ausgewählt aus der Reihe Maltotriose, Maltotetrose, Maltopentose und Maltohexose, 70 bis 100 Gew.-% des Carbohydrats bilden.

6.  Zusammensetzung nach Anspruch 1, enthaltend 4,5 bis 7 g Gesamtprotein, 10 bis 16 g Carbohydrat und 2 bis 3 g Fett in jeweils 100 ml, und wobei ein wasserlösliches Protein mit einem Molekulargewicht im Bereich von 10.000 bis 15.000 35 bis 46 Gew.-% des Gesamtproteins bildet, und ein oder mehrere Oligo(tetra-/penta-)saccharide, ausgewählt aus der Reihe Maltotetrose und Maltopentose, 70 bis 100 Gew.-% des Carbohydrats bilden.

7.  Zusammensetzung nach Anspruch 5, die eine Viskosität von nicht mehr als 5 mPa.s (5 cps) bei 20°C besitzt.

8.  Zusammensetzung nach Anspruch 6, die eine Viskosität von nicht mehr als 5 mPa.s (5 cps) bei 20°C besitzt.

9.  Zusammensetzung nach einem der Ansprüche 1 bis 8, wobei das Protein aus der Reihe Kasein, Natriumkasein, Calciumkasein, enzymatische Abbauprodukte der Kaseine, enzymatisch abgebautes Sojabohnenprotein, enzymatisch abgebautes Weizenprotein und enzymatisch abgebaute Gelatine, ausgewahlt ist, und wobei das Carbohydrat aus der Reihe der Oligo(tetra-/penta-)saccharide, und das Fett aus der Reihe der pflanzlichen Öle ausgewählt ist.

10. Zusammensetzung nach Anspruch 9, wobei das Oligosaccharid Maltotetrose und/oder Maltopentose ist.

11. Verfahren zur Herstellung einer flüssigen Nährmittelzusammensetzung, das die Bereitstellung einer Proteinquelle, die 30 bis 50 Gew.-% eines enzymatisch abgebauten Proteins mit einem Molekulargewicht im Bereich von 800 bis 30.000, und einer Carbohydratquelle, enthaltend 50 bis 100 Gew.-% an ein oder mehreren Oligosacchariden, ausgewählt aus der Reihe Maltotriose, Maltotetrose, Maltopentose und Maltohexose, und einer Fettquelle, und das Mischen und Emulgieren dieser Quellen in Wasser umfaßt, um eine flüssige Zusammensetzung zu erhalten, die 3,5 bis 7 g Gesamtprotein, 5 bis 17 g Carbohydrat und 1 bis 5 g Fett in jeweils 100 ml enthält, und die eine Viskosität bei 20°C von nicht mehr als 7 mPa.s (7 cps) und einen Gesamtkalorienwert von 293 bis 544 kJ (70 bis 130 kcal)/100 ml aufweist.

**Revendications**

1.  Composition nutritive liquide comprenant 3,5 à 7 g de protéine totale, 5 à 17 g d'hydrate de carbone et 1 à 5 g de graisse pour 100 ml, avec une protéine dégradée enzymatiquement soluble dans l'eau ayant un poids moléculaire de 800 à 30.000 représentant de 30 à 50% en poids de cette protéine totale et un ou plusieurs oligosaccharides choisis dans le groupe consistant en maltotriose, maltotétrose, maltopentose et maltohexose représentant de 50 à 100% en poids de cet hydrate de carbone, et cette composition ayant une viscosité ne dépassant pas 7 mPa.s (7 cps) à 20°C et une valeur calorique totale de 293 à 544 kJ (70 à 130 kcal)/100 ml.

2. Composition suivant la revendication 1, qui contient 4,5 à 7 g de protéine totale, 10 à 16 g d'hydrate de carbone et 2 à 3 g de graisse pour 100 ml.

3. Composition suivant la revendication 1, dans laquelle cette protéine soluble dans l'eau ayant un poids moléculaire de 8.000 à 30.000 représente de 35 à 46% en poids de cette protéine totale et un ou plusieurs oligosaccharides choisis dans le groupe consistant en maltotriose, maltotétrose, maltopentose et maltohexose représentent de 70 à 100% en poids de cet hydrate de carbone.

4. Composition suivant la revendication 3, dans laquelle cette protéine soluble dans l'eau a un poids moléculaire de 10.000 à 15.000 et cet hydrate de carbone est un ou plusieurs oligo (tétra-/penta-) saccharides choisis dans le groupe consistant en maltotétrose et maltopentose.

5. Composition suivant la revendication 1, qui contient 4,5 à 7 g de protéine totale, 10 à 16 g d'hydrate de carbone et 2 à 3 g de graisse pour 100 ml et dans laquelle cette protéine soluble dans l'eau ayant un poids moléculaire de 8.000 à 30.000 représente de 35 à 46% en poids de cette protéine totale et un ou plusieurs oligosaccharides choisis dans le groupe consistant en maltotriose, maltotétrose, maltopentose et maltohexose représentent de 70 à 100% en poids de cet hydrate de carbone.

6. Composition suivant la revendication 1, qui contient 4,5 à 7 g de protéine totale, 10 à 16 g d'hydrate de carbone et 2 à 3 g de graisse pour 100 ml et dans laquelle une protéine soluble dans l'eau ayant un poids moléculaire de 10.000 à 15.000 représente de 35 à 46% en poids de cette protéine totale et un ou plusieurs oligo (tétra-/penta-) saccharides choisis dans le groupe consistant en maltotétrose et maltopentose représentent de 70 à 100% en poids de cet hydrate de carbone.

7. Composition suivant la revendication 5, qui a une viscosité ne dépassant pas 5 mPa.s (5 cps) à 20°C.

8. Composition suivant la revendication 6, qui a une viscosité ne dépassant pas 5 mPa.s (5 cps) à 20°C.

9. Composition suivant l'une quelconque des revendications 1 à 8, dans laquelle cette protéine est choisie dans le groupe consistant en caséine, caséinate de sodium, caséinate de calcium, etc., produits de dégradation enzymatique de ces caséines, protéine de soja dégradée enzymatiquement, protéine de blé dégradée enzymatiquement et gélatine dégradée enzymatiquement, cet hydrate de carbone est choisi dans le groupe consistant en oligo (tétra-/penta-) saccharides, et cette graisse est choisie dans le groupe consistant en huiles végétales.

10. Composition suivant la revendication 9, dans laquelle cet oligosaccharide est le maltotétrose et/ou le maltopentose.

11. Procédé pour la production d'une composition nutritive liquide, qui comprend la fourniture d'une source de protéine contenant 30 à 50% en poids d'une protéine dégradée enzymatiquement ayant un poids moléculaire dans la gamme de 800 à 30.000 et d'une source d'hydrate de carbone contenant 50 à 100% en poids d'un ou de plusieurs oligosaccharides choisis dans le groupe consistant en maltotriose, maltotétrose, maltopentose et maltohexose, et une source de graisse, et le mélange et l'émulsification de ces sources dans de l'eau pour donner une composition liquide contenant 3,5 à 7 g de protéine totale, 5 à 17 g d'hydrate de carbone et 1 à 5 g de graisse pour 100 ml, ayant une viscosité à 20°C ne dépassant pas 7 mPa.s (7 cps) et une valeur calorique totale de 293 à 544 kJ (70 à 130 kcal).